Europäisches Patentamt

European Patent Office

Office européen des brevets

(19)

(11) Veröffentlichungsnummer : **0 060 467**
**B1**

(12) **EUROPÄISCHE PATENTSCHRIFT**

(45) Veröffentlichungstag der Patentschrift :
28.08.85

(21) Anmeldenummer : 82101761.3

(22) Anmeldetag : 06.03.82

(51) Int. Cl.⁴ : **D 21 C 3/00, C 12 N 1/22, B 63 B 35/44**

(54) **Herstellung von Cellulose aus Holz oder anderen lignocellulosehaltigen Pflanzen durch mikrobiellen Abbau der Lignocellulose.**

(30) Priorität : 16.03.81 DE 3110117
16.07.81 DE 3128203

(43) Veröffentlichungstag der Anmeldung :
22.09.82 Patentblatt 82/38

(45) Bekanntmachung des Hinweises auf die Patenterteilung : 28.08.85 Patentblatt 85/35

(84) Benannte Vertragsstaaten :
AT DE FR GB IT

(56) Entgegenhaltungen :
DE-A- 2 620 574
DE-A- 2 720 638
DE-A- 2 746 873
ABSTRACT BULLETIN OF THE INSTITUTE OF PAPER CHEMISTRY, Band 42, Nr. 2, Juli 1971, Seite 40 Zusammenfassung Nr. 341 APPLETON, WISCONSIN (US)
A.B.I.P.C., Band 38, Nr. 8, Februar 1968, Seite 566, Zusammenfassung Nr. 5893
A.B.I.P.C., Band 39, Nr. 8, Februar 1969, Seite 651, Zusammenfassung Nr. 6564
PHYSIOL. PLANT, Band 41, 1977 P. ANDER u.a.: "Selective Degradation of Wood Components by White-Rot Fungi", Seiten 239-248

(73) Patentinhaber : Eisenstein, Albin, Dr.-Ing.
Langerstrasse 29
D-4000 Düsseldorf 1 (DE)

Eisenstein, Rubin, Dr.-Ing.
Langerstrasse 29
D-4000 Düsseldorf 1 (DE)

Basler, Adolf
Gunzenbachweg 6
A-5340 St. Gilgen (AT)

(72) Erfinder : Eisenstein, Albin, Dr.-Ing.
Langerstrasse 29
D-4000 Düsseldorf 1 (DE)
Erfinder : Eisenstein, Rubin, Dr.-Ing.
Langerstrasse 29
D-4000 Düsseldorf 1 (DE)
Erfinder : Basler, Adolf
Gunzenbachweg 6
A-5340 St. Gilgen (AT)

(74) Vertreter : Pollmeier, Felix
Patentanwälte Hemmerich-Müller-Grosse-Pollmeier
Eduard-Schloemann-Strasse 47
D-4000 Düsseldorf 1 (DE)

## Beschreibung

Die Erfindung betrifft ein Verfahren zur Gewinnung von Zellstoff in technischem Maßstab, vorzugsweise für die Papier-, und Pappeherstellung, aus Holz oder anderen lignocellulosehaltigen Pflanzen (z. B. Stroh, Schilf, Bagasse usw.) mit Hilfe eines Weißfäulepilzes. Zur Lenkung des erfindungsgemäßen Abbauprozesses gehört die Zugabe geeigneter Enzyme (bevorzugt « Laccasen » und « Peroxydasen ») unter Temperaturerhöhung, damit das eingeleitete Wachstum des Weißfäulepilzes (bevorzugs der « Pleurotus ostreatus ») gestoppt, damit die enzymatische Hydrolyse der Cellulose zu löslichen Zuckern stark eingeschränkt und der alleinige Abbau des Lignins stark gefördert wird.

Die bekanntesten, technisch durchgeführten chemischen Verfahren zur Herstellung von Zellstoff aus Holz oder holzähnlichen Stoffen sind das Sulfit- und das Sulfatverfahren. Beide Verfahren wenden zum Herauslösen des Lignins basische bzw. saure wässrige Lösungen von Chemikalien bei über 100° Celsius und hohem Druck an. Sie sind beide äußerst anlageintensiv und nur bei großen Kapazitäten rentabel, benötigen enorme Mengen an Wasser und Energie, insbesondere zur Verhinderung der Umweltverschmutzung durch das in grossen Mengen anfallende Abwasser und der übelriechenden, schwefelhaltigen Abgase.

Nach weiteren bekannten chemischen Verfahren wird das Lignin unter Anwendung von Druck und/oder hohen Temperaturen mit Hilfe organischer Lösungsmittel, z. B. wässrigen äthylalkoholischen Lösungen, Gemischen aus Dimethylsulfoxid und Äthanolamin, und paraffinischem Mineralöl herausgelöst. Alle diese Verfahren sind wegen ihrer komplizierten Technologie sehr anlageintensiv und erfordern einen hohen Kostenaufwand für Energie.

Der mikrobielle Abbau von Ligno-Cellulose ist durch ein Verfahren bekannt geworden, das in den DE-A-27 46 872 und DE-A-27 46 873 beschrieben ist. Dieses Verfahren kommt als mikrobiologisches Verfahren dem erfindungsgemäßen Verfahren zwar am nächsten, unterscheidet sich jedoch entscheidend in der Zielsetzung, in den Mitteln und im Endprodukt.

Nach diesem Verfahren wird ein Lignocellulose-Substrat von Lignocellulose-Feststoffen in einer Nährlösung mit einem pH-Wert zwischen 4 und 5 mit einer wässrigen Suspension von Sporen des Schimmelpilzes « Chrysosporium pruinosum » inoculiert. Nach einer gewissen Einwirkungszeit wird die Temperatur auf 50 bis 60° Celsius erhöht, um das Wachstum des Pilzes zu beeden. Den weiteren Abbau der Lignocellulose besorgen die während des Wachstums erzeugten Enzyme.

Während das erfindungsgemäße Verfahren das Ziel verfolgt und auch weitgehend erfüllt, in möglichst kurzer Zeit viel Lignin abzubauen und dabei gleichzeitig so wenig wie möglich Cellulose durch enzymatische Hydrolyse in lösbaren Zucker umzusetzen, werden z. B. nach dem bekannten Verfahren nach ca. drei Tagen 20 % Lignin, 40 % Cellulose + Hemicellulosen und 35 % andere organische Bestandteile und nach ca. 12 Tagen (Ende des verstärkten Abbaus von Ligno-Cellulose) 50 % Lignin, 80 % Cellulose + Hemicellulosen und 75 % anderer organischer Bestandteile abgebaut (siehe Fig. 2 der DE-A-27 46 872). Das erfindungsgemäße Verfahren zur technischen Gewinnung von Zellstoff für die Papier- und Pappeherstellung aus Holz oder ligno-cellulosehaltigen Pflanzen geschieht zudem in Zeiträumen von einigen wenigen Stunden, wogegen das bekannte mikrobielle Abbauverfahren zum Abbau derselben Menge Lignin mehrere Tage benötigt.

Der Erfindung liegt nun die Aufgabe zugrunde, die hier angeführten Nachteile der chemischen und des bekannten mikrobiellen Ligninabbau-Verfahrens zu vermeiden.

Diese Aufgabe wird erfindungsgemäß dadurch gelöst, daß man die lignocellulosehaltige Substanz der Einwirkung des Weißfäulepilzes aussetzt, indem man eine Mischung aus zerkleinerten Lignocelluloseteilchen und einer Nährlösung für Weißfäulepilze mit einem pH-Wert von 4,0 bis 6,0 mit Sporen und/oder Zellen eines Weißfäulepilzes inokuliert und einer leichten Zirkulation von Warmluft mit 85 bis 97 % relativer Luftfeuchtigkeit bei 25 bis 35 °C und einer Lichtquelle von 25 Lux aussetzt, dann extracellulär erzeugte, lignin-abbauende Enzyme, bevorzugt Laccasen und/oder Peroxidasen zusetzt und die Lufttemperatur bei gleichbleibender Luftfeuchtigkeit auf 50 bis 60 °C erhöht, und, nachdem der gewünschte Ligninabbau erreicht ist, das erhaltene Faserstoffprodukt wäscht, trocknet und zur völligen Abtötung des Pilzes einer leicht ionisierenden Bestrahlung unterwirft. Das optimale Verhältnis zwischen Fasersubstanz und Nährlösung beträgt etwa 1 : 4, d. h. auf einen Gewichtsteil Fasersubstanz werden 4 Gewichtsteile Nährlösung zugesetzt.

Als Weißfäulepilz hat sich insbesondere der « Pleurotus ostreatus » als geeignet erwiesen, der gemäß einem weiteren Merkmal der Erfindung verwendet wird.

Als Nährlösung wird gemäß einem weiteren Merkmal der Erfindung eine Lösung bestehend aus 1,5 g $K_2HPO_4$ ; 0,5 g $MgSO_4(7\ H_2O)$ ; 10 g $CaCO_3$ ; 2 mg Thiamin HCl ; 1,0 g Bactopepton und 1 000 ml Leitungswasser verwendet, deren Säuregrad, und hierin besteht ebenfalls ein Merkmal der Erfindung, etwa auf ph 5,6 eingestellt ist. Zu achten ist auf eine gleichmäßige Vermischung der Fasersubstanz mit der Nährlösung.

Mit dem Inoculieren beginnt das Wachstum der Weißpilzzellen und die Bildung der Enzyme, die den Abbau der Ligno-Cellulose einleiten. Um den Ligninabbau gegenüber der Hydrolyse der Cellulose zu beschleunigen, werden z. B. extracellulär erzeugte Enzyme, bevorzugt « Laccasen » und « Peroxydasen » dem Substrat in Form einer gleichmäßig aufgesprühten Lösung zugeführt. Nach einer gewissen Zeit muß die Lufttemperatur auf bevorzugt 50-55° Celsius, unter Beibehaltung der etwa 95 % relativen Luftfeuchtigkeit und etwa 25 Lux Beleuchtung, erhöht werden, damit das Pilzwachstum gestoppt wird. Nachdem der gewünschte Ligninabbau erreicht ist, wird das erhaltene Faserstoffprodukt gewaschen. nach der Entwässerung des Faserstoffproduktes erfolgt zunächst eine Trocknung (z. B mittels infraroter Lichtstrahlen) und zum völligen Abtöten des Pilzes eine leicht ionisierende Bestrahlung.

Das Endprodukt ist bei dieser Behandlung von einem großen Teil des Lignins befreit, so wurde bei einer Bearbeitung von Buchenholz nach einem sechsstündigen Abbauprozeß der Gehalt an Lignin von 35 % auf 15 % gesenkt, wobei der gleichzeitig auftretende Celluloseverlust nie größer als 8 % war.

Dem Impfstoff wurde aus Kulturen des « Pleurotus ostreatus » auf Glucose-Agarplatten bei 28° Celsius im Laufe von acht Tagen gezüchtet.

Das beschriebene erfindungsgemäß neuartige mikrobiologische Verfahren hat folgende Vorteile :

a) gegenüber dem Verfahren nach DE-A-27 46 872 und DE-A-27 46 873 :
— beschleunigter Abbau des Lignins
— nur geringfügiger Abbau der Cellulose.

b) gegenüber den chemischen Verfahren :
— geringer Energieverbrauch
— beträchtliche Einsparung an Chemikalien
— keine Umweltverschmutzung
— Möglichkeit der Verwertung des Lignins aus den Abbauprodukten
— Möglichkeit der Errichtung von kleineren Anlagen, die nur geringe Investitionen erfordern und die in industriellem Maßstab wirtschaftlich arbeiten können
— Bleichung des Faserstoffes durch die mit dem Entholzungsprozeß gleichzeitig stattfindende Zerstörung der pigmentierenden Extraktstoffe.

Beispiel

In einem Bunker gelagerte Hackschnitzel werden mittels einer Bunkeraustragsvorrichtung dem Bunker entnommen und gleichmäßig einer Messermühle zugeführt. Die auf dem Rotor peripher angeordneten Messer arbeiten gegen ein oder mehrere stationäre Messer und damit zerkleinern sie die Hackschnitzel. Die Größe der Holzteilchen wird durch die Lochgröße des Siebes, das sich vor der Austragsöffnung befindet, bestimmt. Insbesondere hat sich eine Größe von 5-6 mm bewährt. Die derart in ihrer Größe homogenisierten Holz- oder Pflanzenpartikel werden mittels geeigneter Vorrichtungen entstaubt und anschließend in Mischbottichen mit einer Nährlösung imprägniert.

Das optimale Gewichtsverhältnis von Nährlösung zur festen Fasersubstanz beträgt etwa 4 : 1, d. h. einem Gewichtsteil Fasersubstanz weren vier Gewichtsteile Nährlösung zugesetzt. Bei Arbeiten mit gleichmäßig trockenen Hackschnitzeln, die vor der Einbunkerung schon entstaubt wurden, kann die Vermischung mit der Nährlösung schon in der Messermühle, während des Zerkleinerungsvorganges, erfolgen.

Dieses aus Lignocellulose und Nährlösung bestehende Substrat wird nun mit den Sporen des « Pleurotus ostreatus » inoculiert. Dies kann auf verschiedene Weise erfolgen, z. B. durch Beimischung der Impfsubstanz zur Nährlösung oder durch Besprühen des aufgelockerten und in gleichmäßiger Höhe auf siebartigen Horden gelagerten Substrates. Die Horden können stationär ausgebildet sein, über die zur Inoculierung eine Sprühvorrichtung bewegt wird. Sie können auch in der Art von Förderbändern konstruiert sein. Beispielsweise wird ein aus Draht bestehendes, endloses Siebtuch, das sich über zwei Umkehrrollen, in einem geschlossenen Kanal bewegt, mit dem Substrat belegt, und zwar in einer Dicke von etwa 20-25 cm. An der Stelle, an der das Substrat auf das Band auftritt, wird im Gegenstrom die Impflösung aufgetragen. In dem Kanal herrscht eine relative Luftfeuchtigkeit von etwa 96 % und eine Temperatur von 27° Celsius. Ein Ventilator sorgt für die entsprechende Luftzirkulation und eine Serie von Lampen gewährleisten die erforderliche 25 Lux starke Beleuchtung. Zwecks Beschleunigung des Ligninabbaus werden nach der Inoculierung extrazelluläre Enzyme in Form von Laccasen und Peroxydasen in Lösungen aufgesprüht. Nach einer Zeitspanne, die vom jeweiligen Rohstoff abhängt, wird unter Beibehaltung aller Faktoren des Mediums, die Lufttemperatur auf 50 bis 55° Celsius erhöht. Anschließend wird das delignifizierte Gut gewaschen, entwässert und zur Trocknung durch einen entsprechenden Kanal mit infraroten Strahlen gefördert. Vor Einlagerung und Verpackung wird der Zellstoff kurzfristig einer leichten ionisierenden Bestrahlung ausgesetzt. Das bei der Eindickung anfallende Wasser wird dem Produktionskreislauf nach einer kurzen Ionisierung wieder zugeführt.

Es ist ein Vorteil des erfindungsgemäßen Verfahrens, daß es — wie bereits erwähnt —, auch in kleinen Anlagen in industriellem Maßstab wirtschaftlich betrieben werden kann und gegenüber den bekannten chemischen Verfahren (Sulfit- oder Sulfatverfahren) als Vorteile aufweist :
1. geringer Energieverbrauch,
2. geringere Mindestkapazität (Tagesleistung) für eine rentable Anlage, und damit niedere Investitionen,
3. umweltfreundliche Arbeitsweise, d. h. keine schädlichen Abwässer oder Abgase und keine Ablaugeprobleme.

Somit ist das erfindungsgemäße Verfahren geradezu prädestiniert auf mobilen Fahrzeugen, insbesondere auf Wasserfahrzeugen, installiert zu werden. Nun ist zwar aus den DE-A-26 20 574 und DE-A-27 20 638 ein Wasserfahrzeug bekannt geworden, auf dem Zellstoff nach den bekannten chemischen Verfahren erzeugt wird. Solche Anlagen zur Erzeugung von Zellstoff umfassen zwangsläufig den gesamten Umfang einer kompletten Zellstoff-Fabrik, wie Maschinen zum Zerkleinern, Dämpfen, Entlüften, Kochen, Zerfasern und Entwässern des Rohstoffes, gegebenenfalls auch einer Einrichtung zum Bleichen, Trocknen, zum Pressen und aus Einrichtungen zum Eindicken und Vergasen der Ablaugen und Einrichtungen für die Chemikalienrückgewinnung.

Der rentable Betrieb solch hochgezüchteter chemo-technischen Fabriken ist nur ab einer bestimmten Tageskapazität möglich. Kleine Anlagen können also nicht gebaut werden.

Ein weiterer Nachteil solcher Zellstoffwerke besteht in ihrem relativ hohen Frischwasserbedarf, für das eine aufwendige Wasseraufbereitung erforderlich ist. Weiterhin werden Einrichtungen für die Ablaugenaufbereitung und für die Reinigung der Abwässer benötigt.

Im allgemeinen sind solche Fabriken eine ernste Umweltbelastung.

Beim erfindungsgemäßen Verfahren treten weder umwetschädliche Abwässer oder übelriechende Abluft auf und man benötigt nur einen Bruchteil als rentable Mindestkapazität, auch ist der erforderliche Energieverbrauch klein.

Alls diese Vorzüge sind entscheidende Argumente für die Errichtung mikrobiologischer Zellstoffwerke auf mobilen Fahzeugen und vorwiegend auf Wasserfahrzeugen.

Hierbei ist noch zu beachten, daß die für die Beschleunigung des mikrobiellen Abbauprozeßes erforderliche Wärme aus dem Abdampf oder aus dem Kühlwasser der Fahrzeugkraftanlage gewonnen werden kann. Als Antrieb für die Fertigungsmechanismen kann gleichfalls die Fahrzeugkraftanlage verwendet werden.

Es ist von Vorteil, die für die Erzeugung erforderlichen Weißfäulepilze und Enzyme auf dem mobilen Fahrzeug oder Wasserfahrzeug selbst zu züchten.

## Patentansprüche

1. Verfahren des mikrobiellen Abbaus von Lignocellulose mit Hilfe eines Weißfäulepilzes zur Gewinnung von Cellulose aus Holz oder anderen lignocellulosehaltigen Pflanzenfasermaterialien wie Stroh, Schilf, Bagassen, bei dem man nach Zerkleinerung des Pflanzenfasermaterials und Beimischung einer Nährlösung für Weißfäulepilze mit einem pH-Wert von 4,0 bis 6,0 dem Substrat Sporen und/ oder Zellen eines Weißfäulepilzes inokuliert und bei dem das Substrat einer leichten Zirkulation von Warmluft mit 85 % bis 97 % relativer Luftfeuchtigkeit bei 25 °C bis 35 °C und einer Beleuchtung von 25 Lux aussetzt, bei dem dem Substrat extracellulär erzeugte, ligninabbauende Enzyme, bevorzugt Laccasen und-/oder Peroxidasen, zugesetzt werden, bei dem die Lufttemperatur bei gleichbleibender Luftfeuchtigkeit auf 50 °C bis 60 °C erhöht wird, und bei dem nach Erreichung des gewollten Ligninabbaus das Substrat gewaschen, entwässert, getrocknet und leicht ionisierend bestrahlt wird.

2. Verfahren nach Anspruch 1, dadurch gekennzeichnet, daß Sporen und/oder Zellen des Pleurotus ostreatus inokuliert werden.

3. Verfahren nach einem der Ansprüche 1 oder 2, gekennzeichnet durch die Verwendung einer Nährlösung aus 1,5 g $K_2HPO_4$, 0,5 g $MgSO_4$, 10 g $CaCO_3$, 2 mg Thiamin HCl, 1,0 g Bactopepton gelöst in 1 000 ml Leitungswasser.

4. Verfahren nach Anspruch 3, gekennzeichnet durch die Verwendung einer Nährlösung mit einen pH-Wert von 5,6.

5. Verfahren nach einem der vorhergehenden Ansprüche, gekennzeichnet durch ein Substrat aus einem Gewichtsteil Fasersubstanzen und 4 Gewichtsteilen Nährlösung.

6. Verfahren nach einem der Ansprüche 1 bis 5, dadurch gekennzeichnet, daß die Gewinnung von Zellstoff durch mikrobiellen Abbau der Lignine auf mobilen Fahrzeugen, vorzugsweise auf Wasserfahrzeugen, stattfindet.

7. Verfahren nach Anspruch 6, dadurch gekennzeichnet, daß die für die Erzeugung erforderlichen Weißfäulepilze und Enzyme auf dem mobilen Fahrzeug oder Wasserfahrzeug selbst gezüchtet werden.

8. Verfahren nach Anspruch 6 oder 7, dadurch gekennzeichnet, daß die für die Beschleunigung des mikrobiellen Abbauprozeßes erforderliche Wärme bei Wasserfahrzeugen aus dem Abdampf der Antriebsturbine und/oder aus dem Kühlwasser der Diesel- oder Gasmotoren der Kraftanlage gewonnen wird.

9. Verfahren nach einem der Ansprüche 6 bis 8, dadurch gekennzeichnet, daß die für die Erzeugung erforderliche mechanische Energie von der Kraftstation des mobilen Fahrzeuges geliefert wird.

## Claims

1. Process of microbiological decomposition of lignocellulose with the help of a white rot fungus for the extraction of cellulose from wood or other lignocellulose-containing fibrous plant material such as straw, reed, bagasse, in which, after comminution of the fibrous plant material and admixture of a nutrient solution for white rot fungus having a pH value of 4.0 to 6.0, the substrate is innoculated with spores and/or cells of a white rot fungus, and the substrate is exposed to a gentle circulation of hot air with relative humidity of 85 % to 97 % at 25 °C to 35 °C and an illumination level of 25 Lux, to the substrate is added extracellular produced, lignin decomposing enzymes, preferably « laccasses » and/or peroxidases, the air temperature is raised to 50° to 60 °C with the humidity remaining constant, and after reaching the desired lignin decomposition the substrate is washed, dewatered, dried and subjected to gentle ionising radiation.

2. Process according to Claim 1, characterized in that spores and/or cells of Pleurotus ostreatus are used for innoculation.

3. Process according to one of Claims 1 or 2, characterized by the use of a nutrient solution of 1.5 g $K_2HPO_4$, 0.5 g $MgSO_4$, 10 g $CaCO_3$, 2 mg Thiamin HCl, 1.0 g Bactopepton dissolved in 1 000 ml tap water.

4. Process according to Claim 3, characterized by the use of a nutrient solution with a pH value of 5.6.

5. Process according to one or more of the preceding Claims, characterized by a substrate of one part by weight fibrous material and four parts by weight nutrient solution.

6. Process according to one of Claims 1 to 5, characterized in that the extraction of cellulose by microbial decomposition of lignin takes place on vehicles, preferably on water craft.

7. Process according to Claim 6, characterized in that the white rot fungus and enzymes necessary for the production are cultured on the vehicle or water craft itself.

8. Process according to Claim 6 or 7, characterized in that the heat necessary for the acceleration of the microbial extraction process is obtained, on water craft, from the exhaust steam of the drive turbine and/or from the cooling water of the diesel or petrol engine of the power plant.

9. Process according to one of Claims 6 to 8, characterized in that the mechanical energy necessary for the production is delivered by the power source of the mobile vehicle.

**Revendications**

1. Procédé de dégradation microbienne de la lignocellulose au moyen d'une moisissure blanche pour l'obtention de cellulose à partir du bois ou d'autres matières végétales contenant de la lignocellulose, telles que paille, roseaux, bagasses, dans lequel après le broyage de la matière fibreuse végétale et le mélange avec une solution nutritive pour les moisissures blanches à un pH de 4,0 à 6,0, on inocule au substrat des pores et/ou des cellules d'une moisissure blanche et on soumet le substrat à une légère circulation d'air chaud à 85-97 % d'humidité relative à l'air à 25-35 °C et à un éclairement de 25 lux, on ajoute au substrat des enzymes dégradant la lignine, produites par voie extracellulaire, de préférence des laccases et/ou des peroxydases, on élève la température de l'air à 50-60 °C en maintenant constante l'humidité de l'air, et après avoir atteint la dégradation voulue de la lignine, on lave le substrat, on le déshydrate, on le sèche et on le traite par irradiation légèrement ionisante.

2. Procédé selon la revendication 1, caractérisé en ce que l'on inocule des spores et/ou des cellules de « Pleurotus ostreatus ».

3. Procédé selon l'une des revendications 1 ou 2, caractérisé en ce que l'on utilise une solution nutritive constituée par 1,5 g de $K_2HPO_4$, 0,5 g de $MgSO_4$, 10 g de $CaCO_3$, 2 mg de thiamine, HCl, 1,0 g de bactopeptone, dissous dans 1 000 ml d'eau du robinet.

4. Procédé selon la revendication 3, caractérisé en ce que l'on utilise une solution nutritive ayant un pH de 5,6.

5. Procédé selon l'une des revendications précédentes, caractérisé en ce que le substrat consiste en 1 partie en poids de substances fibreuses et 4 parties en poids de solution nutritive.

6. Procédé selon l'une des revendications 1 à 5, caractérisé en ce que l'obtention de cellulose par dégradation microbienne des lignines a lieu sur des véhicules mobiles, de préférence sur des bateaux.

7. Procédé selon la revendication 6, caractérisé en ce que les moisissures blanches et les enzymes nécessaires pour la production sont élevées ou produites sur le véhicule mobile ou le bateau lui-même.

8. Procédé selon la revendication 6 ou 7, caractérisé en ce que la chaleur nécessaire pour l'accélération du processus microbien de dégradation est récupérée à partir de la vapeur résiduaire de la turbine d'entraînement et/ou de refroidissement des moteurs Diesel ou à gaz de la centrale électrique dans le cas des bateaux.

9. Procédé selon l'une des revendications 6 à 8, caractérisé en ce que l'énergie mécanique nécessaire pour la production est fournie par la centrale électrique du véhicule mobile.